(19) Europäisches Patentamt
European Patent Office
Office européen des brevets

(11) EP 2 659 877 A2

(12) **EUROPEAN PATENT APPLICATION**

(43) Date of publication:
**06.11.2013 Bulletin 2013/45**

(21) Application number: **13154310.0**

(22) Date of filing: **07.02.2013**

(51) Int Cl.:
***A61K 8/36*** *(2006.01)*
***A61K 8/365*** *(2006.01)*
***A61K 8/42*** *(2006.01)*
***A61Q 17/04*** *(2006.01)*
***A61Q 19/00*** *(2006.01)*
***A61Q 19/06*** *(2006.01)*
***A61Q 19/08*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.03.2012 US 201213414346**

(71) Applicant: **Evonik Industries AG**
**45128 Essen (DE)**

(72) Inventors:
- **Meyer, Jürgen**
  **45134 Essen (DE)**
- **Hameyer, Peter**
  **45138 Essen (DE)**
- **Howe, Anna M**
  **Moseley, VA Virginia 23120 (US)**
- **Spohrer, Maria L**
  **Glen Allen, VA 23059 (US)**
- **Paez, Angela Dawn**
  **Prince George, VA Virginia 23875 (US)**

(54) **Cosmetic formulation with a dry skin feel**

(57) The instant invention relates to cosmetic compositions with improved dry skin feel characteristics. In particular, the present invention relates to cosmetic formulations comprising certain amidoamines used in combination with fatty acids and selected further acids.

EP 2 659 877 A2

## Description

The instant invention relates to cosmetic compositions with improved dry skin feel characteristics. In particular, the present invention relates to cosmetic formulations comprising certain amidoamines used in combination with fatty acids and selected further acids.

Background

Cosmetic formulations which are useful in skin conditioning and leave a good dry skin feel are usually based on the addition of quaternized compounds.
Quaternized compounds need to be prepared in a chemical process that makes use of quaternization agents such as methylchloride or dimethylsulfate. The toxicological and environmental profile of such quaternization agents is not preferred for cosmetic applications in times in which consumers are asking for more natural and eco-friendly ingredients for cosmetics.
It is an objective of the instant invention to provide for alternative cosmetic formulations with good skin conditioning performance and a good dry skin feel comparable to those achieved with quaternized compounds.

Detailed Description of the Invention

It has surprisingly been found that cosmetic formulations according to the present invention contribute to a solution of the above mentioned objective.
It is an advantage of the instant invention that the cosmetic compositions can be distributed on the skin very easily.
A further advantage of the instant invention is that the cosmetic compositions are very stable.
Yet another advantage of the instant invention is that the cosmetic compositions are non sticky.
Another advantage of the invention is that it allows the preparation of robust emulsion systems with a relatively low use level of emulsifiers.
Moreover, these emulsions systems are flexible as they can be prepared using a wide variety of different cosmetic oils.
The present invention is directed to a cosmetic formulation containing

A) is at least one substance of general formula I

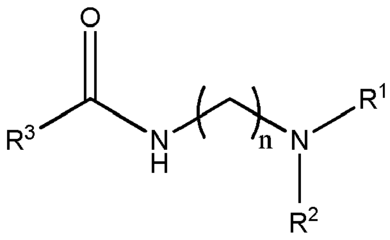
general formula I

wherein

$R^1$ and $R^2$ independently from each other, equal or unequal, are chosen from linear or branched, saturated or unsaturated alkyl residues with 1 to 22 carbon atoms, preferably linear or branched, saturated alkyl residues with 1 to 3 carbon atoms, especially preferably $-CH_3$, and $-CH_2CH_3$,
$R^3$ is a linear or branched, saturated or unsaturated alkyl residue with 9 to 27 carbon atoms, more preferred, linear and saturated alkyl residue with 11 to 23 carbon atoms, even more preferred, linear and saturated alkyl residue with 17 to 21 carbon atoms and n = 1 - 6, preferably 2 - 4, most preferred 3,

B) is at least one fatty acid chosen from linear or branched, saturated or unsaturated, with 18 to 32 carbon atoms, even more preferred. linear and saturated with 18 to 22 carbon atoms and
C) is at least one acid with a pKa in the range of -10 to 6, more preferred, -7 to 5.8 even more preferred -7 to 4.8.

The general formula I depicted above also includes protonated structures, especially at the amine-nitrogen (the one bound to $R^1$ and $R^2$).
In a preferred embodiment, the inventive formulation contains a substance of general formula I, with $R^1$ and $R^2$ being $-CH_3$, and $-CH_2CH_3$ and $R^3$ being a linear and saturated alkyl residue with 17 to 21 carbon atoms and n being

3, even more preferred, component A) is selected from the group consisting of stearamidopropyldimethylamine and behenamidopropyldimethylamine.

It is of advantage, when at least a part of component A) is protonated at the nitrogen atom of the amine group. This usually occurs due to the neutralization of component A) by the acidic component C). Therefore the ratio of component A) to C) has an influence on the degree of neutralization (protonation) of component A).
The molar ratio X of component A) and B) in a given composition as used herein is calculated by the formula

$$X = \frac{n_C}{n_A} = \frac{\frac{m_C}{M_C}}{\frac{m_A}{M_A}}$$

with $n_C$, $n_A$ being the molar quantities of the components C and A, respectively,
$m_C$, $m_A$ being the net weight of components C and A, respectively in the formulation in [g], and $M_C$, $M_A$ being the molecular weights of components C and A, respectively, in [g/mol]. In a preferred embodiment, the cosmetic formulation is characterized in, that it has a pH in the range of from 3 to 7 and that the molar ratio X is > 0.2.
Further preferred are cosmetic formulations characterized in, having a pH in the range of from 3.2 to 6 and the molar ratio X being > 0.5.
Even further preferred are cosmetic formulations characterized in, having a pH in the range of from 3.8 to 5.5 and the molar ratio X being > 0.7.
The pH is to measured at 25 °C.

Component A) preferably is contained in the inventive formulation in an amount of 0.1 wt.% to 10 wt.%, more preferably of 0.6 wt.% to 5 wt.%, even more preferably 1 to 2 wt%, wherein in the weight percent refer to the complete formulation.

In a further preferred embodiment, the cosmetic formulation is characterized in that component B) is selected from the group consisting of
palmitic acid, margaric acid, stearic acid, nonadecylic acid, arachidic acid, heneicosylic acid, behenic acid, tricosylic acid, lignoceric acid, pentacosylic acid, cerotic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid;
with palmitic acid, stearic acid and behenic acid being most preferred.

Component B) preferably is contained in the inventive formulation in an amount of 0.6 wt.% to 10 wt.%, more preferably of 1 wt.% to 6 wt.%, even more preferably 2 to 4 wt%, wherein in the weight percent refer to the complete formulation.

In a further preferred embodiment, the cosmetic formulation is characterized in that component C) is selected from the group consisting of
acetic acid, adipic acid, ascorbic acid, benzoic acid, butyric acid, enol pyrivic acid, glutaric acid, glycolic acid, ketovaleric acid, lactic acid, beta lactic acid, methylbenzoic acid, pentanoic acid, phenylacetic acid, propionic acid, pyrrolcarboxylic acid, succinic acid, citric acid, hydrochloric acid, sulfuric acid, phosphoric acid, sulphonic acid, gluconic acid, phytic acid and vinylacetic acid,
with acetic acid, lactic acid, citric acid, hydrochloric and phosphoric acid being most preferred.

Component C) preferably is contained in the inventive formulation in an amount sufficient to neutralize component A) in the inventive formulation in the range of 20 wt.% to 100 wt.%, more preferably 50 wt.% to 95 wt.% and even more preferably 70 wt.% to 90 wt.% referring to the whole amount of component A)

In a further preferred embodiment, the cosmetic formulation is characterized in that the formulation further contains at least one polyol like for example glycerin, diglycerin, ethoxylated glycerin, propoxylated glycerin, propylene glycol, dipropylene glycol, polypropylene glycol, polyethylene glycol, sorbitol, hydroxylpropyl sorbitol, hexylene glycol, 1,3-butylene glycol and 1,2,6 hexanetriol, and blends thereof and the like, wherein glycerin and propylene glycol are the most preferred polyols to be contained.

Preferably the formulation according to the invention has a pH in the range of 2.0 to 6.0, more preferably of 3.0 to 5.0 and even more preferably of 3.5 to 4.5 measured at 25°C.

The cosmetic formulation according to the invention preferably is an emulsion, preferably an o/w-emulsion.

Preferably the cosmetic formulation according to the instant invention contains an oil-phase of from 10 wt.% to 95 wt.%, more preferably from 15 wt.% to 70 wt.%, even more preferably from 20 wt.% to 40 wt.% referring to the complete formulation.

Preferably the cosmetic formulation according to the instant invention does not contain any quarternized components.

In a further preferred embodiment, the cosmetic formulation according to the invention contains

component A) in an amount of 0.1 wt.% to 10 wt.%, more preferably of 0.6 wt.% to 5 wt.%, even more preferably 1 to 2 wt%,

component B) in an amount of 0.6 wt.% to 10 wt.%, more preferably of 1 wt.% to 6 wt.%, even more preferably 2 to 4 wt%,

component C) in an amount of 0.001 wt.% to 5 wt.%, more preferably of 0.01 wt.% to 2 wt.%, even more preferably 0.05 to 1 wt%,

wherein in the weight percent refer to the complete formulation.

In a further preferred embodiment, the cosmetic formulation is characterized in that the formulation has a viscosity of 500 - 200,000 mPas, more preferably of 1000 - 100000 mPas, even more preferably of 5000 to 50,000 mPas, measured at a shear rate of $10s^{-1}$ at 25 °C.

Examples:

*Example 1: Sensory profile of compositions according to the invention*

| RAW MATERIAL | Comparative example 1 | Example 1 | Example 2 |
|---|---|---|---|
| D.I. Water | ad 100 | ad 100 | ad 100 |
| Glycerin | 5.00 | 5.00 | 5.00 |
| Cetearyl Alcohol | 2.00 | 2.00 | 2.00 |
| VARISOFT® TA 100[1)] | 2.00 | 0.00 | 0.00 |
| TEGO® AMID S 18[2)] | 0.00 | 2.00 | 0.00 |
| TEGO® AMID S 22[2)] | 0.00 | 0.00 | 2.00 |
| Lactic Acid (85%) | 0.54 | 0.54 | 0.54 |
| Ethylhexyl Palmitate | 6.00 | 6.00 | 6.00 |
| Stearic Acid | 3.00 | 3.00 | 3.00 |
| Mineral Oil | 5.00 | 5.00 | 5.00 |
| Petrolatum | 4.50 | 4.50 | 4.50 |
| Microcare PM34 | 0.80 | 0.80 | 0.80 |
| pH | 4.50 | 4.50 | 4.50 |

[1)] Distearyldimonium Chloride (Evonik Industries AG)
[2)] Stearamidopropyl Dimethylamine (Evonik Industries AG)
[3)] Behenamidopropyl Dimethylamine (Evonik Industries AG)
[4)] Methylparaben, Ethylparaben, Propylparaben, Phenoxyethanol (Thor Specialties Inc.)

The sensory profile of the formulations has been assessed by a panel consisting of 15 trained panelists. The attributes "tackiness", "slip", "absorption", "oiliness" and "waxiness" have been rated immediately after application and after 5 minutes. The rating was done on a scale from 0 (attribute not present) to 10 (attribute very strongly present). On this scale, a difference of 2 can be described as a noticeable sensory difference.

Table 1: Sensory panel rating for example formulations versus comparison example based on a quaternized emulsifier.

| Formulation | Directly after application | | | | | After 5 minutes | | | | |
|---|---|---|---|---|---|---|---|---|---|---|
| | Tackiness | Oiliness | Absorption | Waxiness | Slip | Tackiness | Oiliness | Absorption | Waxiness | Slip |
| Comparison Example 1 | 4 | 3 | 5 | 5 | 5 | 3 | 3 | 7 | 5 | 1 |
| Example 1 | 4 | 3 | 4 | 5 | 5 | 3 | 3 | 7 | 5 | 2 |
| Example 2 | 4 | 4 | 5 | 6 | 4 | 3 | 3 | 7 | 6 | 2 |

As illustrated in table 1, formulation examples according to the present invention based either on Stearamidopropyl Dimethylamine or Behenamidopropyl Dimethylamine are matching the cationic target profile in a very convincing way.
This example shows that a "cationic" skin feel can be achieved according to the invention without the need to use quaternized ingredients.

*Example 2: Stability of compositions according to the invention*

To show that it is far from trivial to arrive at the formulation at the instant invention and how critical the specific choice of component B) is, the following formulations were prepared:

| PHASE | RAW MATERIAL | F1 | F2 | F3 | F4 | F5 | CF1 | CF2 |
|---|---|---|---|---|---|---|---|---|
| B | D.I. Water | ad 100 | ad 100 | ad 100 | ad 100 | ad 100 | ad 100 | ad 100 |
| | Glycerin | 5.00 | 5.00 | 5.00 | 5.00 | 5 | 5.00 | 5.00 |
| | Lactic Acid (85%) | 0.54 | | | | 0.54 | 0.54 | 0.54 |
| | Citric Acid (100%) | | 0.34 | | | | | |
| | HCL (36%) | | | 0.54 | | | | |
| | Sulfuric Acid (100%) | | | | 0.52 | | | |
| A | Cetearyl Alcohol | 2.00 | 2.00 | 2.00 | 2.00 | 2.00 | 2.00 | 2.00 |
| | TEGO® AMID S 18[1)] | 2.00 | 2.00 | 2.00 | 2.00 | 2.00 | 2.00 | 2.00 |
| | Ethylhexyl Palmitate | 6.00 | 6.00 | 6.00 | 6.00 | 6.00 | 6.00 | 6.00 |
| | Stearic Acid | 3.00 | 3.00 | 3.00 | 3.00 | | | |
| | Lauric Acid | | | | | | 3.00 | |
| | Myristic Acid | | | | | | | 3.00 |
| | Behenic Acid | | | | | 3.00 | | |
| | Mineral Oil | 5.00 | 5.00 | 5.00 | 5.00 | 5.00 | 5.00 | 5.00 |
| | Petrolatum | 4.50 | 4.50 | 4.50 | 4.50 | 4.50 | 4.50 | 4.50 |
| C | Methylparaben, Ethylparaben, Propylparaben, Phenoxyethanol (Thor Specialties Inc.) | 0.80 | 0.80 | 0.80 | 0.80 | 0.80 | 0.80 | 0.80 |

("F"=formulation according to the invention; "CF" comparative formulation)
[1)]Stearamidopropyl Dimethylamine (Evonik Industries AG)

The formulations were prepared by combining the components of phase A and heating them to 70 °C, combining the components of phase B and heating them to 70 °C, adding phase B to phase A without stirring, subsequently stirring intensely at 1400rpm for 5 minutes with a magnetic stirrer and reducing stirrer speed to 900 rpm while cooling to room temperature.
The storage stability was tested at 50 °C.
All formulations according to the invention F1 to F5 show no phase separation and are stable after several weeks and months whereas formulation CF1 and CF2 show severe instability after one week.

*Further formulation examples:*

Light Sun Care Soft Cream O/W with Skin Firming Properties

| PHASE | RAW MATERIAL | % |
|---|---|---|
| A | D.I. Water | ad 100 |
| | Glycerin | 2.0 |
| | Lactic Acid (85%) | 0.54 |
| | Polysorbate 80 | 0.3 |
| | Sodium Lactate; Sodium PCA; Glycine; Fructose; Urea; Niacinamide; Inositol; Sodium Benzoate; Lactic Acid | 1.0 |
| | Allantoin | 0.1 |
| B | Polysorbate 80 | 0.3 |
| | Cetearyl Alcohol | 1.0 |
| | TEGO® AMID S 18[1)] | 2.0 |
| | Phenoxyethyl Caprylate | 8.0 |
| | Glyceryl Stearate | 2.0 |
| | Octocrylene | 7.0 |
| | Benzophenone-3 | 5.0 |
| | Ethylhexyl Methoxycinnamate | 3.0 |
| | Methyl Anthranilate | 4.0 |
| | Polyamide-4 (Arizona Chemical Company) | 0.2 |
| | Caprylic/Capric Triglyceride; Xymenynic Acid | 2.0 |
| C | Titanium Dioxide; Trimethyoxycaprylylsilane | 4 |
| | Xanthan Gum | 0.2 |
| D | Preservative | q.s. |

[1)]Stearamidopropyl Dimethylamine (Evonik Industries AG)

Cationic O/W Sun Cream

| PHASE | RAW MATERIAL | % |
|---|---|---|
| A | D.I. Water | ad 100 |
| | Glycerin | 3.0 |
| | Lactic Acid (85%) | 0.54 |
| | Creatine | 0.5 |

(continued)

| PHASE | RAW MATERIAL | % |
|---|---|---|
| B | Stearyl Alcohol | 1.0 |
| | TEGO® AMID S 18[1)] | 2.0 |
| | C12-15 Alkyl Benzoate | 5.0 |
| | Cetyl Ricinoleate | 1.0 |
| | Diethylhexyl Carbonate | 3.0 |
| | Triisostearin | 1.0 |
| | Glyceryl Stearate | 1.5 |
| | Butyl Methoxydibenzoylmethane | 2.0 |
| | Ethylhexyl Methoxycinnamate | 7.0 |
| | Methyl Anthranilate | 4.0 |
| C | Preservative | q.s. |
| [1)]Stearamidopropyl Dimethylamine (Evonik Industries AG) | | |

Energizing Anti-Aging Cream

| PHASE | RAW MATERIAL | % |
|---|---|---|
| A | D.I. Water | ad 100 |
| | Lactic Acid (85%) | 0.54 |
| | Tetrapeptide-21; Glycerin; Butylene Glycol; water | 2.0 |
| | Creatine | 0.5 |
| | Sodium Chloride | 0.5 |
| B | Microcrystalline Wax | 1.2 |
| | Isohexadecane | 9.5 |
| | TEGO® AMID S 18[1)] | 2.0 |
| | Caprylic/Capric Triglyceride | 6.5 |
| | Cetearyl Ethylhexanoate | 4.0 |
| C | Preservative | q.s. |
| [1)]Stearamidopropyl Dimethylamine (Evonik Industries AG) | | |

Anti-Cellulite Lotion

| PHASE | RAW MATERIAL | % |
|---|---|---|
| A | D.I. Water | ad 100 |
| | Glycerin | 3.0 |
| | Lactic Acid (85%) | 0.54 |
| | Caffeine | 1.0 |
| | Xanthan Gum | 0.2 |
| B | TEGO® AMID S 18[1)] | 2.0 |
| | Diethylhexyl Carbonate | 3.5 |
| | Ethylhexyl Palmitate | 1.10 |
| | Cetearyl Isononanoate | 10.0 |
| | Caprylic/Capric Triglyceride; Xymenynic Acid | 2.0 |
| C | Sodium Hydroxide (10% in water) | q.s. |
| Z | Preservative | q.s. |
| [1)]Stearamidopropyl Dimethylamine (Evonik Industries AG) | | |

3 in 1 O/W Lotion with Protection, Regeneration and Preventation Properties

| PHASE | RAW MATERIAL | % |
|---|---|---|
| A | D.I. Water | ad 100 |
| | Glycerin | 3.0 |
| | Lactic Acid (85%) | 0.54 |
| | Polysorbate 80 | 0.2 |
| | Creatine | 0.3 |
| | Panthenol | 0.5 |
| | Ceteareth-25; Glycerin; Cetyl Alcohol; Behenic Acid; Cholesterol; Ceramide NP; Ceramide NS; Ceramide EOS;CeramideEOP;Ceramide AP; Caprooyl Phytosphingosine; Caprooyl Sphingosine | 5.0 |
| | Sodium Hydroxide (10% in water) | 0.7 |
| | Allantoin | 0.1 |
| B | TEGO® AMID S 18[1)] | 2.0 |
| | Bis-PEG/PPG-16/16 PEG/PPG-16/16 Dimethicone; Caprylic/Capric Triglyceride | 1.0 |
| | Diethylhexyl Carbonate | 5.50 |
| | Ethylhexyl Palmitate | 4.0 |
| | Octyldodecanol | 4.0 |
| | Ethylhexyl Stearate | 1.6 |
| | Xanthan Gum | 0.2 |
| | Tocopheryl Acetate | 0.5 |
| C | Preservative | q.s. |
| [1)]Stearamidopropyl Dimethylamine (Evonik Industries AG) | | |

Spot Evening Hand Cream

| PHASE | RAW MATERIAL | % |
|---|---|---|
| A | D.I. Water | ad 100 |
| | Glycerin | 5.5 |
| | Lactic Acid (85%) | 0.54 |
| B | D.I. Water | q.s. |
| | Tetrapeptide-30; Glycerin | 2.5 |
| | Sodium Ascorbyl Phosphate | 1.5 |
| | Urea | 2.5 |
| | Sodium Bisulfate | 0.1 |
| | D.I. Water | 10.0 |
| C | Cetearyl Alcohol | 2.0 |
| | TEGO® AMID S 18[1)] | 2.0 |
| | Caprylic/Capric Triglyceride | 3.0 |
| | Ethylhexyl Stearate | 3.0 |
| | Cetyl Dimethicone | 2.5 |
| | Dimethicone | 1.5 |
| | Stearic Acid | 2.0 |
| D | Preservative | q.s. |
| [1)]Stearamidopropyl Dimethylamine (Evonik Industries AG) | | |

Cell Protection Body Lotion with Anti-Aging Properties

| PHASE | RAW MATERIAL | % |
|---|---|---|
| A | D.I. Water | ad 100 |
| | Glycerin | 3.0 |
| | Lactic Acid (85%) | 0.54 |
| | Creatine | 0.5 |
| | Curcuma Longa (Turmeric) Root Extract | 0.5 |
| B | TEGO® AMID S 18[1)] | 2.0 |
| | Ethylhexyl Stearate | 7.6 |
| | Decyl Oleate | 5.7 |
| | Glyceryl Stearate | 0.5 |
| | Stearic Acid | 0.7 |
| C | Preservative | q.s. |
| [1)]Stearamidopropyl Dimethylamine (Evonik Industries AG) | | |

Dermal Filler Cream

| PHASE | RAW MATERIAL | % |
|---|---|---|
| A | D.I. Water | ad 100 |
| | Glycerin | 5 |
| | Lactic Acid (85%) | 0.54 |
| | Sodium Chloride | 0.05 |
| B | Cetyl Alcohol | 3.75 |
| | TEGO® AMID S 18[1)] | 2.0 |
| | Isopropyl Palmitate | 4.25 |
| | Dimethicone | 0.4 |
| | Aqua; Ethylhexyl Stearate; Sodium Hyaluronate Crosspolymer; Polyglyceryl-4 Diisostearate/ Polyhydroxystearate/Sebacate; Sodium Isostearate | 3.0 |
| | Petrolatum | 4.55 |
| C | Preservative | q.s. |
| [1)]Stearamidopropyl Dimethylamine (Evonik Industries AG) | | |

O/W Basic Cationic Emulsion

| PHASE | RAW MATERIAL | % |
|---|---|---|
| A | D.I. Water | ad 100 |
| | Glycerin | 5 |
| | Lactic Acid (85%) | 0.54 |
| | Sodium Chloride | 0.05 |
| B | Cetyl Alcohol | 3.75 |
| | TEGO® AMID S 18[1)] | 2.0 |
| | Isopropyl Palmitate | 4.25 |
| | Dimethicone | 0.4 |
| | Petrolatum | 4.55 |
| C | Preservative | q.s. |
| [1)]Stearamidopropyl Dimethylamine (Evonik Industries AG) | | |

O/W Caring Cationic Emulsion

| PHASE | RAW MATERIAL | % |
|---|---|---|
| A | D.I. Water | ad 100 |
| | Glycerin | 5 |
| | Lactic Acid (85%) | 0.54 |
| | Sodium Chloride | 0.05 |
| B | Cetearyl Alcohol | 5 |
| | TEGO® AMID S 18[1)] | 2.0 |
| | Cetearyl Ethylhexanoate; Isopropyl Myristate | 2.0 |
| | Mineral Oil | 5.0 |
| | Petrolatum | 5.0 |
| C | Preservative | q.s. |
| [1)]Stearamidopropyl Dimethylamine (Evonik Industries AG) | | |

While the present invention has been particularly shown and described with reference to the preferred embodiments, it will be readily appreciated by those of ordinary skill in the art that various changes and modifications may be made without departing from the spirit and scope of the invention. It is intended that the present invention be understood to cover the disclosed embodiments, those alternatives which have been discussed and all equivalents thereto.

**Claims**

1. A cosmetic formulation comprising:

A) is at least one substance of general formula I

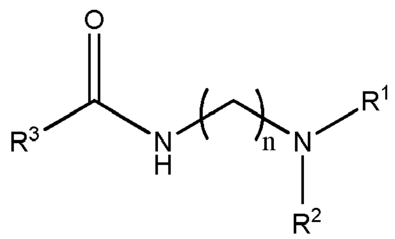

general formula I

wherein

$R^1$ and $R^2$ are independently, equal or unequal, linear or branched, saturated or unsaturated alkyl residues with 1 to 22 carbon atoms,
$R^3$ is a linear or branched, saturated or unsaturated alkyl residue with 9 to 27 carbon atoms, and
n=1-6,

B) is at least one fatty acid which is linear or branched, saturated or unsaturated with 18 to 32 carbon atoms, and
C) is at least one acid with a pKa in the range of -10 to 6.

2. The cosmetic formulation according to claim 1 or 2 wherein component A) is selected from the group consisting of stearamidopropyldimethylamine and behenamidopropyldimethylamine.

3. The cosmetic formulation according to any of the preceding claims, wherein said formulation has a pH in the range of from 3 to 7 and that the molar ratio X is > 0.2, wherein X is determined by

$$X = \frac{n_C}{n_A} = \frac{\frac{m_C}{M_C}}{\frac{m_A}{M_A}}$$

with $n_C$, $n_A$ being the molar quantities of the components C and A, respectively, $m_C$, $m_A$ being the net weight of components C and A, respectively, in the formulation in [g], and
$M_C$, $M_A$ being the molecular weights of components C and A, respectively, in [g/mol].

4. The cosmetic formulation according to any of the preceding claims, wherein component B) is selected from the group consisting of
palmitic acid, margaric acid, stearic acid, nonadecylic acid, arachidic acid, heneicosylic acid, behenic acid, tricosylic acid, lignoceric acid, pentacosylic acid, cerotic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid, docosahexaenoic acid.

5. The cosmetic formulation according to any of the preceding claims, wherein component C) is selected from the group consisting of
acetic acid, adipic acid, ascorbic acid, benzoic acid, butyric acid, enol pyrivic acid, glutaric acid, glycolic acid, ketovaleric acid, lactic acid, beta lactic acid, methylbenzoic acid, pentanoic acid, phenylacetic acid, propionic acid, pyrrolcarboxylic acid, succinic acid, citric acid, hydrochloric acid, sulfuric acid, phosphoric acid, sulphonic acid, gluconic acid, phytic acid and vinylacetic acid.

6. The cosmetic formulation according to any of the preceding claims, wherein the formulation has a pH in the range of 2.0 to 6.0.

7. The cosmetic formulation according to any of the preceding claims, wherein the formulation is an o/w-emulsion.

8. The cosmetic formulation according to any of the preceding claims, wherein the formulation further contains at least one polyol.

9. The cosmetic formulation according to any of the preceding claims, wherein the formulation comprises an oil-phase of from 10 wt.% to 95 wt.%, of the complete formulation.

10. The cosmetic formulation according to any of the preceding claims, wherein the formulation does not contain any quarternized components.

11. The cosmetic formulation according to any of the preceding claims, wherein the formulation comprises component A) in an amount of 0.1 wt.% to 10 wt.%, component B) in an amount of 0.6 wt.% to 10 wt.%, and component C) in an amount of 0.001 wt.% to 5 wt.%, each of the complete formulation.

12. The cosmetic formulation according to any of the preceding claims, wherein the formulation has a viscosity of 500 - 200,000 mPas, measured at a shear rate of $10s^{-1}$.

13. The cosmetic formulation of any of the preceding claims wherein $R^1$ and $R^2$ are independently
$-CH_2$ and $-CH_2CH_3$.

14. The cosmetic formulation of any of the preceding claims, wherein $R^3$ is linear and saturated with
17 to 21 carbon atoms.

15. The cosmetic formulation of any of the preceding claims wherein n is 2 - 4.